(19) Europäisches Patentamt
European Patent Office
Office européen des brevets

(11) Veröffentlichungsnummer: **0 398 340 B1**

(12) **EUROPÄISCHE PATENTSCHRIFT**

(45) Veröffentlichungstag der Patentschrift: **05.01.94**

(51) Int. Cl.[5]: **C07C 233/18**, C07D 295/12, C07C 47/263, C07C 47/277, C07C 49/24, C07C 49/225

(21) Anmeldenummer: **90109372.4**

(22) Anmeldetag: **17.05.90**

(54) **Ceramidderivate und ihre Verwendung als Inhibitoren der Sphingolipidsynthese.**

(30) Priorität: **17.05.89 DE 3916072**

(43) Veröffentlichungstag der Anmeldung:
**22.11.90 Patentblatt 90/47**

(45) Bekanntmachung des Hinweises auf die Patenterteilung:
**05.01.94 Patentblatt 94/01**

(84) Benannte Vertragsstaaten:
**AT CH DE FR GB LI**

(56) Entgegenhaltungen:
**EP-A- 0 146 810**
**EP-A- 0 293 006**

**JOURNAL OF THEORETICAL BIOLOGY, Band 63, November/Dezember 1976, Seite 117-124, Academic Press, London, New York, San Francisco; S. KANG et al: "Conformation of N-formyl-1,3-dihydroxy-delta-pentene, a Model Compound of Sphingomyelin"**

(73) Patentinhaber: **THERA - Patent Verwaltungs-GmbH**
**Griesberg 2**
**D-82229 Seefeld(DE)**

(72) Erfinder: **Wieland, Felix, Prof. Dr.**
**Bergstrasse 80**
**D-6900 Heidelberg(DE)**

(74) Vertreter: **Müller, Bernhard, Dr. H. Schmidt & B. Müller Patentanwälte**
**Graf-Toerring-Strasse 45**
**D-82229 Seefeld (DE)**



Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

INTERNATIONAL JOURNAL OUANTUM CHEMISTRY: OUANTUM BIOLOGY SYMPOSIUM, Nr. 3, 1976, Seite 29-38; B. PULLMAN et al: "Ouantum-Mechanical Studies on the Conformation of Sphingomyelins"

PATENT ABSTRACTS OF JAPAN, Band 10, Nr. 43 (C-329)(2100); 20. Februar 1986; & JP-A-60190745

PATENT ABSTRACTS OF JAPAN, Band 12, Nr. 59 (C-478)(2906), 23. Februar 1988; & JP-A-62 207 247

TETRAHEDRON LETTERS, Band 27, Nr. 4, Seite 481-484, Pergamon Press Ltd.; R. R. SCHMIDT et al: "Synthesis of D-Erythro-Sphingosines"

LIEBIGS ANNALEN DER CHEMIE, 1988, Seite 663, 664; P. ZIMMERMANN et al: "Synthese von erytro-Sphingosinen über die Azidoderivate"

**Beschreibung**

Die Erfindung betrifft Ceramidderivate und ihre Verwendung als Inhibitoren der Sphingolipidsynthese.

Unter Sphingolipiden werden Lipide verstanden, bei denen statt des bei den Phospholipiden vorliegenden Diacetylglyzerins ein Aminodialkohol, nämlich das Sphingosin der nachstehenden Formel

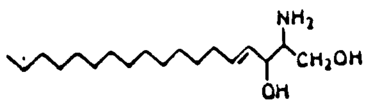

enthalten ist.

Zu den natürlich vorkommenden Sphingolipiden gehören die sogenannten Ceramide, bei denen an der Aminogruppe ein Fettsäurerest vorliegt. Unter "Ceramid" selbst versteht man im allgemeinen N-Palmitylsphingosin der nachstehenden Formel:

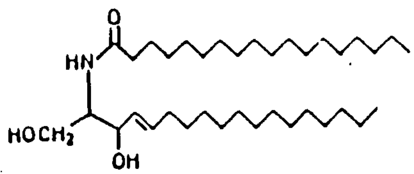

Wie andere Sphingolipide erfüllen Ceramide wichtige Aufgaben beim Aufbau von Zellmembranen. Die Untersuchung von Zellmembranen und ihrer Transportmechanismen stellt ein biochemisches Forschungsgebiet von allgemeinem Interesse dar. Auch von der medizinischen Seite her besteht ein starkes Interesse an derartigen Transportmechanismen, da es zahlreiche Stoffwechselerkrankungen gibt, die mit einer Störung des Sphingolipidstoffwechsels verbunden sind, wobei im allgemeinen eine Anreicherung spezieller Sphingolipide im Körper erfolgt.

Aufgabe der Erfindung ist es, Inhibitoren der Sphingolipidsynthese in Säugetierzellen bereitzustellen, mit denen spezielle Untersuchungen von Stoffwechsel- und Transportmechanismen von Sphingolipiden durchgeführt werden können.

Erfindungsgemäss wurde festgestellt, dass Ceramide, deren Kohlenstoffketten sowohl im Sphingosinteil als auch im Fettsäureteil verkürzt sind, überraschenderweise eine Hemmwirkung auf die Sphingolipidsynthese in Säugetierzellen besitzen.

Gegenstand der Erfindung sind Ceramidderivate der allgemeinen Formel

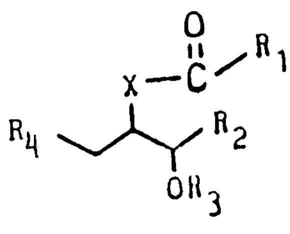

in der die einzelnen Reste folgende Bedeutungen haben:

X = NH oder $CH_2$,
$R_1$ = H oder $C_{1-11}$-Alkyl,
$R_2$ = $C_{2-9}$-Alkenyl, wobei die Doppelbindung in $\alpha$-Stellung zur $OR_3$-Gruppe vorliegt.
$R_3$ = H, $C_{1-6}$-Alkyl oder $C_{1-6}$-Acyl und
$R_4$ = $C_{1-6}$-Alkoxy oder Morpholino.

In einer bevorzugten Gruppe von Verbindungen der Erfindung haben die einzelnen Reste folgende Bedeutungen:

X = NH,
$R_1$ = $C_{1-8}$-Alkyl,

$R_2$ = $C_{3-6}$-Alkenyl,
$R_3$ = H, Methyl oder Acetyl und
$R_4$ = $OCH_3$ oder Morpholino.

Dabei befindet sich die Doppelbindung des Alkenylrests in α-Stellung zu dem die $OR_3$-Gruppe tragenden C-Atom und weist vorzugsweise die trans-Konfiguration auf, jedoch sind auch die cis-Verbindungen von besonderem Interesse, wie nachstehend noch dargelegt wird.

Eine bevorzugte Verbindung ist die Verbindung der Formel:

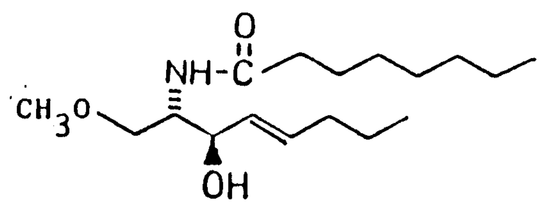

Eine weitere bevorzugte Verbindung ist (2S, 3R, 4E)-2-Decanoylamino-4-decen-1-morpholino-3-ol der folgenden Formel:

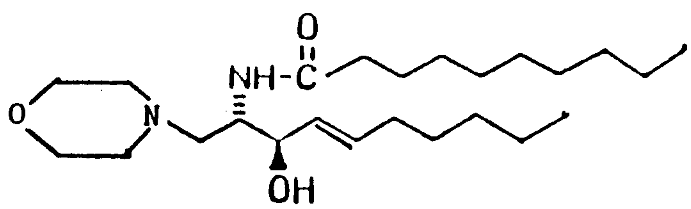

Gegenstand der Erfindung ist ferner die Verwendung von Ceramidderivaten der allgemeinen Formel:

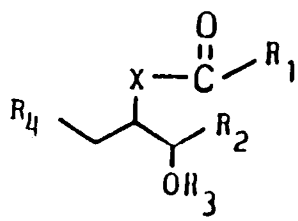

in der die einzelnen Reste die folgenden Bedeutungen haben
X = NH oder $CH_2$
$R_1$ = H oder $C_{1-11}$-Alkyl
$R_2$ = $C_{2-9}$-Alkyl oder $C_{2-9}$-Alkenyl
$R_3$ = H, $C_{1-6}$-Alkyl oder $C_{1-6}$-Acyl und
$R_4$ = OH, $C_{1-6}$-Alkoxy oder Morpholino
als Inhibitoren der Sphingolipidsynthese.

Eine erfindungsgemäß vorzugsweise verwendete Verbindung ist (2S,3R,4E)-2-Octanoylamino-4-octen-1,3-diol (nachstehend als Verbindung 6 bezeichnet) der folgenden Formel:

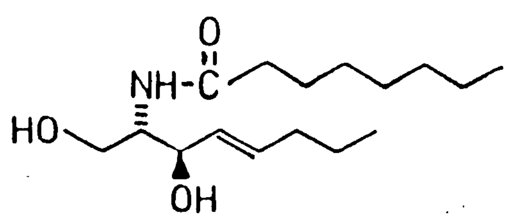

Der Mechanismus der Hemmwirkung der Verbindungen der Erfindung ist zwar nicht bekannt, jedoch ist folgende spekulative Erklärung hierfür denkbar. Wird verkürztes Ceramid einem Zellkulturmedium zugesetzt, so kann diese Verbindung dank ihrer amphiphilen Eigenschaften praktisch ungehindert durch die Plasmamembran der Zellen ins Cytosol und von dort durch Organellenmembranen ins Innere von Zellkompartimenten diffundieren. Dort, z.B. im Golgi-Apparat, kann sie dann als Substrat für die Synthese von verkürzten Sphingolipiden, z.B. Cerebrosiden und Sphingomyelinen, dienen. Diese verkürzten Sphingolipide können nun wegen ihrer polaren Kopfgruppe nicht mehr frei durch Membranen permeieren, sondern werden (wahrscheinlich auf dem Weg des biosynthetischen Transports endogener Sphingolipide) zur Plasmamembran transportiert. Im Gegensatz zu den normalen Sphingolipiden bleiben die kurzen Sphingolipide wegen ihrer verkürzten hydrophoben Schwänze jedoch nicht in der Plasmamembran verankert, sondern werden an das umgebende Medium (oder Blut) abgegeben. Auf diesem Weg wird die Synthese endogener, physiologischer Lipide unterdrückt.

Die cis-Verbindungen sind deshalb von Interesse, weil sie nach ersten Erkenntnissen nicht glycosyliert, wohl aber zu Sphingomyelin umgesetzt werden. Dies stellt eine interessante Spezifität dar.

Die vorgenannte Verbindung mit der Methylgruppe als $R_4$ ist als potentieller Inhibitor der Glycosphingolipidsynthese von besonderem Interesse.

Die Herstellung der erfindungsgemässen verkürzten Ceramide erfolgt analog der kürzlich beschriebenen Synthese der natürlich vorkommenden Ceramide. Diese bekannte Synthese verläuft über eine leicht aus D-Galaktose zugängliche 2,4-di-O-geschützte D-Threose, die ein Zwischenprodukt für die D-Erythrosphingosin-Synthese darstellt, wobei diese Synthese über eine trans-selektive Wittigreaktion, die Einführung einer Azidgruppe an der ungeschützten Hydroxylgruppe und eine anschliessende Azidreduktion verläuft (vgl. Richard R. Schmidt und P. Zimmermann, Tetrahedron Letters, Bd. 27 (1986), S.481-484).

Nachstehend wird die Erfindung anhand von Beispielen näher erläutert. Dabei beschreibt das Beispiel 1 anhand des folgenden Reaktionsschemas die Herstellung der erfindungsgemäß verwendeten Verbindungen Nr. 6 und 7, während in den Beispielen 2 und 3 die Ergebnisse von biochemischen Untersuchungen unter Verwendung der Verbindung Nr. 6 bzw. der Morpholinoverbindung wiedergegeben sind.

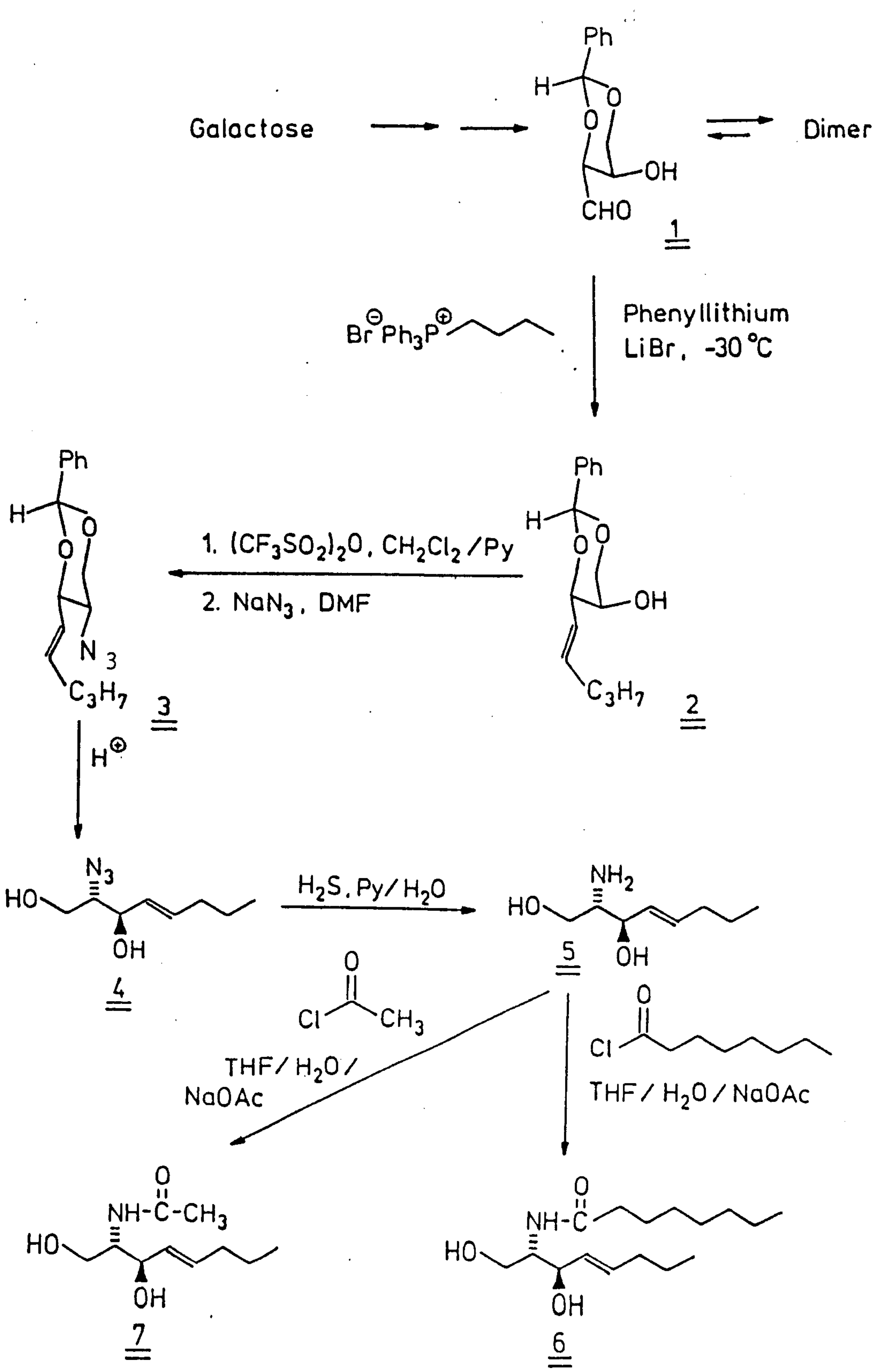


Beispiel 1

Allgemeine Angaben bezüglich der Methoden und Materialien:
Die verwendeten Lösungsmittel wurden nach üblichen Methoden gereinigt. -

$^1$H-NMR-Spektren: Bruker WM250 Cryospec und Jeol JNM-GX400. Tetramethylsilan als innerer Standard - Säulenchromatographie: Kieselgel 60 (Fa. Merck; Korngrösse 0,063-0,2 mm). - Flashchromatographie: Kieselgel 60 (Fa. Merck; Korngrösse 0,040-0,063 mm), - Mitteldruckchromatographie: LiChroprep Si 60 (Fa. Merck; Korngrösse 15-25 µm). - Dünnschichtchromatographie: DC-Plastikfolien, Kieselgel 60 $F_{254}$ (Fa. Merck; Schichtdicke 0,2 mm). - Schmelzpunkte wurden im Kupferblock bestimmt; sie sind unkorrigiert. - Drehwerte: Perkin-Elmer 241 MC; 1-dm-Küvette.

2,4-O-Benzyliden-D-threose (1)*

30,0 g (111 mMol) 4,6-O-Benzyliden-D-galactose** werden in ca. 1,2 Liter Phosphatpuffer von pH 7,6 mit 50,0 g (257 mMol) Natriumperjodat umgesetzt.
Ausb. 20,0 g (85%), DC (Kieselgel; Toluol/Ethanol, 3:1) $R_f = 0,64$.

(2R,3R,4E)-1,3-O-Benzyliden-4-octen-1,2,3-triol (2)

52,0 g (130 mMol) Butyltriphenylphosphoniumbromid werden unter Stickstoff in 600 ml wasserfreiem, stickstoffgesättigtem Toluol suspendiert. Phenyllithium, welches aus 6,5 g (940 mMol) Lithium und 74 g (470 mMol) Brombenzol in 200 ml wasserfreiem Diethylether hergestellt worden ist, wird ohne weitere Reinigung zugetropft. Gleichzeitig wird das Gemisch auf -30 °C abgekühlt. Anschliessend werden 21,6 g (104 mMol) (1) in 150 ml wasserfreiem THF unter Schutzgasbedingungen tropfenweise hinzugegeben. Danach wird mit 150 ml Methanol und 250 ml Wasser versetzt. Die organische Phase wird nach üblichen Methoden aufgearbeitet. Ausb. 11,6 g (45%) Schmp. 42-43 °C, DC (Kieselgel: Petrolether/Essigester, 9:1) $R_f = 0,16$. $[\alpha]_D^{20} = 6,5$ (C = 1,00, $CHCl_3$).
$^1$H-NMR (250MHz, $CDCl_3$): $\delta$ = 7,54-7,31 (2 m, 5H, $C_6H_5$), 5,86 (dt, 1H, CH=CH-$CH_2$, J=6,6 Hz, J=15,6 Hz), 5,65 (dd, 1H, CH=CH-$CH_2$, J=6,1Hz, J=17 Hz), 5,61 (s, 1H, CH-Ph), 4,39 (d, 1H, O-CH-CH=, J=6,1Hz), 4,22 (dd, 1H, O-$CH_2$, J=1,8 Hz, J=12 Hz), 4,06 (dd, 1H, O-$CH_2$, J=1,4 Hz, J=12 Hz), 3,52 (dd, 1H, CH-OH, J=1,2 Hz, J=10,4 Hz), 2,71 (d, 1H, OH, J=10,4 Hz), 2,04 (m, 2H, CH=CH-$CH_2$), 1,43 (sext., 2H, $CH_2$-$CH_3$, J=7,3 Hz), 0,91 (t, 3H, $CH_3$, J=7,3 Hz).

(2S,3R,4E)-2-Azido-1,3-O-benzyliden-4-octen-1,3-diol (3)

11,0 g (44 mMol) der Verbindung (2) werden unter Feuchtigkeitsausschluss in 100 ml Dichlormethan und 10 ml Pyridin gelöst. Bei -15 °C werden unter Argon-Schutzgas 15,5 g (55 mMol) Trifluormethansulfonsäureanhydrid langsam zugetropft. Anschliessend verdünnt man mit 100 ml wasserfreiem Dimethylformamid und gibt 11,4 g (176 mMol) Natriumazid hinzu. Nach 2 Stunden kräftigem Rühren bei Raumtemperatur arbeitet man wie üblich auf.
Ausb. 7,2 g (60%) farbloses Öl, DC (Kieselgel:Petrolether/Essigester, 9:1)
$R_f = 0,68$, $[\alpha]_D^{22} = -21,1$ (c = 1,00, $CHCl_3$).
$^1$H-NMR (250MHz, $CDCl_3$): $\delta$ = 7,56-7,34 (2 m, 5H, $C_6H_5$), 5,95(dt, 1H, CH=CH-$CH_2$, J=6,8 Hz, J=15,6 Hz), 5,58 (dd, 1H, CH=CH-$CH_2$, J=7,3 Hz, J=15,6 Hz), 5,49 (s, 1H, CH-Ph), 4,34 (dd, 1H, O-$CH_2$, J=5,0Hz, J=11 Hz), 4,06 (dd, 1H, O-CH-CH=, J=7,9 Hz, J=8,5 Hz), 3,61 (dd, 1H, O-$CH_2$, J=11,4 Hz, J=11,4 Hz), 3,52-3,42 (m, 1H, CH-$N_3$), 2,10 (m, 2H, CH=CH-$CH_2$), 1,48 (sext., 2H, $CH_2$-$CH_3$, J=7,3 Hz), 0,93 (t, 3H, $CH_3$, J=7,3 Hz).

(2S,3R,4E)-2-Azido-4-octen-1,3-diol (4)

6,5g (35 mMol) (3) werden in 100 ml wasserfreiem Methanol gelöst und nach Zusatz von 200 mg (1,05 mMol) p-Toluolsulfonsäuremonohydrat 48 Stunden bei Raumtemperatur gerührt. Anschliessend wird durch Zugabe von festem $NaHCO_3$ neutralisiert und wie üblich aufgearbeitet.
Ausb. 4,08 g (63%) farbloses Öl, DC (Kieselgel: Dichlormethan/Methanol, 95:5) $R_f = 0,31$, $[\alpha]_D^{20} = -48,03$ (c = 1,00, $CHCl_3$).
$^1$H-NMR (250 MHz, $CDCl_3$): $\delta$ = 5,82(dt, 1H, CH=CH-$CH_2$, J=6,7 Hz, J=15,6 Hz), 5,55 (dt, 1H, CH=CH-$CH_2$, J=7,2 Hz, J=15,4 HZ), 4,25 (m, 1H, O-CH), 3,78 (m, 2H, O-$CH_2$), 3,53-3,47 (m, 1H, CH-$N_3$), 2,48 (m,

* P.Zimmermann und R.R.Schmidt, Lieb.Ann.Chem., (1988), S.663-667;

** E.G.Gros und V.Deulofeu, J.Org.Chem., Bd.29 (1964), S.3647-3654.

2H, OH), 2,05 (q, 2H, CH=CH-$CH_2$, J=7,6 Hz), 1,43 (sext., 2H, $CH_2$-$CH_3$, J=7,3 Hz), 0,92 (t, 3H, $CH_3$, J=7,3 Hz).

(2S,3R,4E)-2-Amino-4-octen-1,3-diol (5)

In 100 ml eines $H_2S$-gesättigten Gemisches aus Wasser/Pyridin (1:1) werden 1,5 g (8 mMol) der Verbindung 4 bei Raumtemperatur 52 Stunden gerührt. Die Aufarbeitung erfolgt nach den üblichen Methoden.
Ausb. 1,2 g (93%) harzige Substanz, DC (Kieselgel: Chlorform/Methanol, 1:1), $R_f$=0.19, $[\alpha]_D^{20}$ = +2,8 (c= 10, $CHCl_3$).
$^1$H-NMR(400 MHz, $CDCl_3$): $\delta$ =5,73 (dt, 1H, CH=CH-$CH_2$, J=6,7 Hz, J=15,4 Hz), 5,45 (dd, 1H, CH=CH-$CH_2$), J=7,1 Hz, J=15,4 Hz), 4,03 (t, 1H, O-CH, J=6,3 Hz), 3,68-3,64 (m, 2H, O-$CH_2$), 3,17 (breites m, 4H, 2OH, $NH_2$), 2,81-2,77 (m, 1H, OH-$NH_2$), 2,03 (q, 2H, CH-$CH_2$, J=7,3 Hz), 1,40 (sext., 2H, $CH_2$-$CH_3$, J=7,3 Hz), 0,91 (t, 3H, $CH_3$, J=7,5 Hz).
Die Verbindung (5) lässt sich leicht mit methanolischer HCl quantitativ in das Hydrochlorid-Derivat überführen.

(2S,3R,4E)-2-Octanoylamino-4-octen-1,3-diol (6)

78,6 mg (0,49 mMol) (5) werden in einem Zweiphasensystem aus 12 ml THF und 12 ml 50%iger wässriger Natriumacetatlösung unter kräftigem Rühren mit 80,3 mg (0,49 mMol) Octansäurechlorid umgesetzt. Nach einer Reaktionszeit von 12 Stunden wird wie üblich aufgearbeitet.
Ausb. 114 mg (82%). Schmp. 65-66 °C, DC (Kieselgel:Chloroform/Methanol 9:1), $R_f$=0,45.
$^1$H-NMR (250 MHz, $CDCl_3$): $\delta$ =6,32 (d, 1H, NH, J=7,0 Hz), 5,78 (dt, 1H, CH=CH-$CH_2$, J=6,7 Hz, J=15,6 Hz), 5,53 (dd, 1H, CH=CH-$CH_2$, J=6,4 Hz, J=15,4 Hz), 4,31 (m, 1H, CH-N), 3,96-3,88 (m, 2H, O-$CH_2$, O-CH), 3,72-3,68 (m, 1H, O-$CH_2$), 3,07 (m, 2H, OH), 2,23 (t, 2H, NH-CO-$CH_2$), 2,05 (q, 2H, CH=CH-$CH_2$, J=7,3 Hz), 1,66-1,61 (m, 2H, NH-CO-$CH_2$-$CH_2$), 1,40 (sext., 2H, $CH_2$-$CH_3$, J=7,3 Hz), 1,19-1,37 (m, 8H, $CH_2$), 0,90 (t, 3H, $CH_3$, J=7,3 Hz), 0,88 (t, 3H, $CH_3$, J=7,3 Hz).

(2S,3R,4E)-2-Acetylamino-4-octen-1,3-diol (7)

Verbindung (7) wird analog dem Verfahren wie für (6) beschrieben durch Verwendung von 117,9 mg (0,74 mMol) der Verbindung (5) und 58,1 mg (0,74 mg) Acetylchlorid hergestellt.
Ausb. 119,1 mg (80%) farbloses Öl, DC (Kieselgel: Chloroform/Methanol, 8:2) $R_f$=0,54.
$^1$H-NMR (400 MHz, $CDCl_3$): $\delta$ =6,40 (d, 1H, NH, J=7,0 Hz), 5,78 (dt, 1H, CH=CH-$CH_2$, J=6,8 Hz, J=15,4 Hz), 5,56 (dd, 1H, CH=CH-$CH_2$, J=6,4 Hz, J=15,4 Hz), 4,32 (m, 1H, CH-N), 4,03-3,81 (m, 2H, O-$CH_2$, O-CH), 3,79-3,64 (m, 1H, O-$CH_2$), 3,17-2,97 (m, 2H, OH), 2,08-2,03(m, 5H, CH=CH-$CH_2$, CO-$CH_3$), 1,41 (sext., 2H, $CH_2$-$CH_3$, J=7,3 Hz), 0,90 (t, 3H, $CH_3$, J=7,3 Hz).

(2S,3R,4E)-2-Decanoylamino-4-decen-1-morpholino-3-ol (8)

Die Herstellung dieser Verbindung erfolgt analog zur Herstellung der Verbindung (6). Abweichend vom vorstehenden Reaktionsschema wird die Verbindung (4) mit Methansulfonylchlorid umgesetzt. Die dabei erhaltene Verbindung mit einer Mesylgruppe in der 1-Stellung ergibt nach Umsetzung mit Morpholin die entsprechende 1-Morpholinoverbindung, die dann analog zum Reaktionsschema zur Titelverbindung umgesetzt wird.

Beispiel 2

Zur Untersuchung der biochemischen Wirkung der Verbindungen der Erfindung wird eine Zellkultur von Ovarialzellen des chinesischen Hamsters (Chinese Hamster Ovary (CHO)-Zellen) in einem Alpha-Medium der Fa. Seromed (0,2% $NaHCO_3$, ohne Antibiotika, ohne fötales Kälberserum oder mit 7,5% fötalem Kälberserum) mit der Verbindung Nr. 6 (3H-markiert; 2,3-Di-$^3$H-octansäurederivat) in einer Konzentration von 5 mikromolar versetzt und 180 Minuten bei 37 °C inkubiert. Im Anschluss daran wurden nach Zentrifugation sowohl das Medium als auch das Zellextrakt (erhalten durch Extraktion der Zellen mit $CH_3OH/H_2O$ = 1:1) einer dünnschichtchromatographischen Untersuchung unter folgenden Bedingungen unterworfen: 25µm Kieselgel Merck; Laufmittel Butanon/Aceton/$H_2O$ = 30:3:5).

Die Analyse der entstandenen Produkte erfolgte durch

a) Verdauungsversuche mit spezifischen, abbauenden Enzymen, d.h. Spingomyelinase, β-Glukosidase und Endoglycoceramidase ( Enzyme der Firmen Böhringer Mannheim und Genzyme) (Fig. 1) und
b) massenspektroskopische Untersuchung (Fast Atom Bombardment Methode).

Die Ergebnisse der Verdauungsversuche sind im Autoradiogramm von Fig. 1 wiedergegeben. Dabei haben die einzelnen Spuren folgende Bedeutungen:

1) 2,3-Di-$^3$H-octansäure
2) eingesetztes 2,3-Di-$^3$H-octanoyl-$C_8$-sphingosin (verkürztes, radioaktives Ceramid $^3$H-GCA)
3) Medium der Inkubation der CHO-Zellen für 180 min bei 37°C mit 100 µCi $^3$H-GCA/ml (= ca. 10 µM). 5 µl des Mediums wurden aufgetragen; Fluorographie für 24 h auf Kodak X-Omat-Film.
4) Probe wie in 3), für 20 h bei 23°C gehalten (als Vergleich zur Verdauung mit Sphingomyelinase).
5) Probe wie in 3) und 4), jedoch für 20 h bei 23°C mit Sphingomyelinase aus menschlicher Plazenta (Sigma) inkubiert.

Die Fast Atom Bombardment-Analyse ergab folgende $M^+$- bzw. $M+H^+$-Peaks (Unter $C_8$-Verbindungen werden hier die Verbindungen verstanden, die 8 C-Atome im Sphingosinteil und im Fettsäurerest aufweisen):

| Verbindung | Peak | |
|---|---|---|
| $C_8$-Ceramid (MG 285) | $M+H^+$ | 286 |
| $C_8$-Sphingomyelin (MG 451) | $M^+$ | 451 |
| $C_8$-Glukosylceramid (MG 447) | $M+H^+$ | 448 |

Die Geschwindigkeit der Bildung und des Transports von $C_8$-Sphingomyelin und $C_8$-Glukosylceramid konnte gemessen werden (Fig. 2). Die Versuchsdurchführung hierzu war folgende.

2 ml CHO-Zellen in α-MEM-Medium ohne foetales Kälberserum wurden mit 10 nMol (150 µCi) $^3$H-GCA ($C_8$) versetzt und bei 37°C im Zellinkubator gerührt. Zu den angegebenen Zeiten wurden 100 µl-Aliquotanteile entnommen und 30 sec bei 0°C und 10000 g zentrifugiert. Das überstehende Medium wurde jeweils abpipettiert (in Figg. 2A und 2B als "Media" bezeichnet). Die Zellniederschläge wurden mit $CH_3OH/H_2O$ = 1:1 (100 µl) extrahiert und wieder zentrifugiert. Medien und Extrakte wurden (jeweils 5 µl) auf Kieselgel 60 (Merck) in Butanon/Aceton/$H_2O$ = 30:3:5 chromatographiert. Die Platten wurden getrocknet, und die radioaktiven Flecken wurden in einem Berthold Linear TLC Radioanalyzer quantitativ bestimmt. Die Zählausbeute bei $^3$H mit dieser Methode beträgt ca 1%, bezogen auf cpm in Lösung. "Cts" gibt die gesamtem gemessenen Impulse an (in diesem Versuch war die Messzeit 5 min pro Spur).

Aus Figg. 2A und 2B ist ersichtlich, dass in der Zelle ein schneller Anstieg der synthetisierten Verbindungen auf einen Plateauwert erfolgt, der über Stunden in der Zelle aufrechterhalten bleibt. Im Medium treten die Verbindungen erst nach einer Verzögerungsphase auf und werden dann linear akkumuliert. Diese Befunde stimmen mit der vorstehend vorgetragenen Hypothese überein, das das verkürzte Ceramid in die Zelle permeieren kann, dort umgesetzt wird und dann aus der Zelle heraustransportiert wird. Auf diese Weise lässt sich eine kompetitive in vivo-Hemmung der Sphingolipidsynthese erreichen.

## Beispiel 3

## Hemmung der Glucosylceramid-Synthese durch (2S,3R,4E)-2-Decanoylamino-4-decen-1-morpholino-3-ol.

Die Synthese der Sphingolipide erfolgt im Golgi Apparat.

### A. Hemmung in intakten Golgi Membranen.

Golgi Membranen (20µl, 60 µg Membranprotein) wurden in TRIS-gepufferter Kochsalzlösung pH 7,4 45 min bei 37°C in Gegenwert steigenden Mengen des Hemmstoffs (0, 5, 10, 25, 50, 100, 250 µM Endkonzentration) inkubiert. Die Aktivität der Sphingolipidsynthese dieser Ansätze wurde durch gleichzeitige Zugabe von $^3$H-Ceramid $C_8C_8$ ((2S,3R,4E)-2-Octanoylamino-4-octen-1,3-diol) und Quantifizierung der Produkte Sphingomyelin $C_8C_8$ und Glucosylceramid $C_8C_8$ nach Dünnschichtchromatographie gemessen.

### B. Hemmung in intakten Zellen

Chinese Hamster Ovary (CHO)-Zellen in Suspension wurden mit den angegeben Konzentrationen an Hemmstoff für 15 min bei 37°C vorinkubiert. Die Aktivität der Sphingolipidsynthese wurde nach den

angegbenen Zeiten in Medien und Zellextrakten (Zellextrakt = Niederschlag nach Zentrifugation des Aliquots extrahiert mit 50 % wässrigem Isopropanol, Volumen entsprechend dem Volumen des Aliquots) wie unter A angegeben bestimmt.

Die Ergebnisse der Versuche A und B sind in Figuren 3 bis 5 gezeigt. Fig. 3 zeigt, daß eine Inhibitorzugabe bis 250 µM die Sphingomyelinsynthese nicht beeinflusset, während die Glucosylceramidsynthese auf weniger als 15 % abnimmt. Somit wird (2S, 3R, 4E)-2-Decanoylamino-4-decen-1-morpholino-3-ol als spezifischer Inhibitor der Glucosyceramidsynthese angesehen.

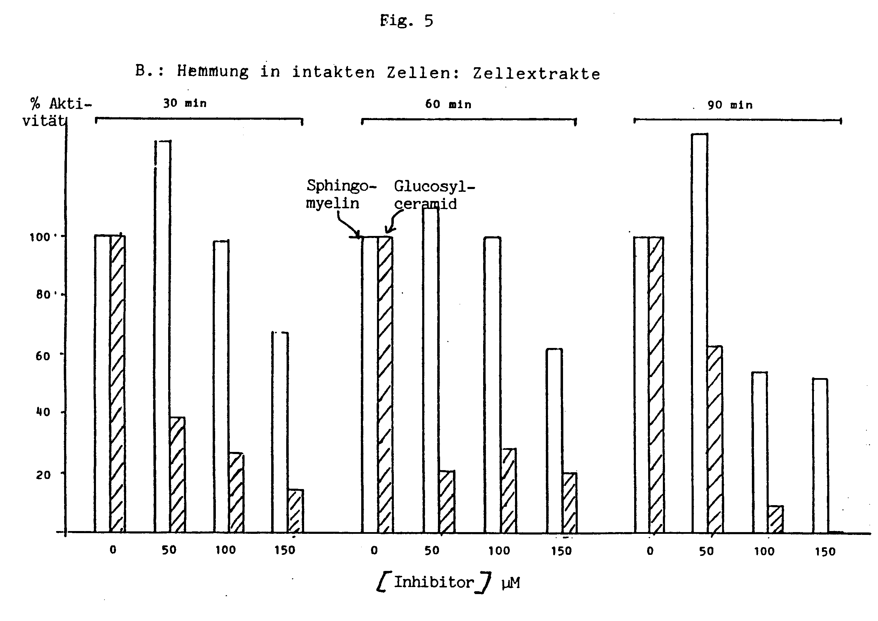

Figg. 4 und 5 zeigen, dass die Bildung von Sphingomyelin $C_8C_8$ (offene Säulen) geringer als die Bildung von Glucosylceramid (schraffierte Säulen) gehemmt wird. Das Ergebnis ist in Zellen ausgeprägter als im Zellmedium. Die Ergebnisse in intakten Zellen sind nicht so eindeutig wie in intakten Golgi Membranen.

**Patentansprüche**

1. Ceramidderivate der allgemeinen Formel

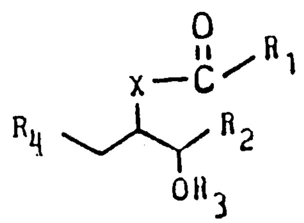

in der die einzelnen Reste folgende Bedeutungen haben:

X = NH oder $CH_2$,
$R_1$ = H oder $C_{1-11}$-Alkyl,
$R_2$ = $C_{2-9}$-Alkenyl, wobei die Doppelbindung in α-Stellung zur $OR_3$-Gruppe vorliegt,
$R_3$ = H, $C_{1-6}$-Alkyl oder $C_{1-6}$-Acyl und
$R_4$ = $C_{1-6}$-Alkoxy oder Morpholino.

2. Verbindungen nach Anspruch 1, dadurch gekennzeichnet, dass die einzelnen Reste folgende Bedeutungen haben:

X = NH,
$R_1$ = $C_{1-8}$-Alkyl,
$R_2$ = $C_{3-6}$-Alkenyl
$R_3$ = H, Methyl oder Acetyl und
$R_4$ = $OCH_3$ oder Morpholino.

3. Verbindungen nach Anspruch 1 oder 2, dadurch gekennzeichnet, dass die Doppelbindung des Alkenylrestes im Rahmen von $R_2$ die trans-Konfiguration besitzt.

4. Verbindungen nach Anspruch 1 oder 2, dadurch gekennzeichnet, dass die Doppelbindung des Alkenylrestes im Rahmen von $R_2$ die cis-Konfiguration besitzt.

5. Verbindung nach Anspruch 1 mit folgender Formel:

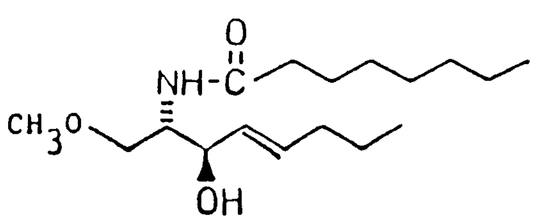

6. Verbindung nach Anspruch 1 mit folgender Formel:

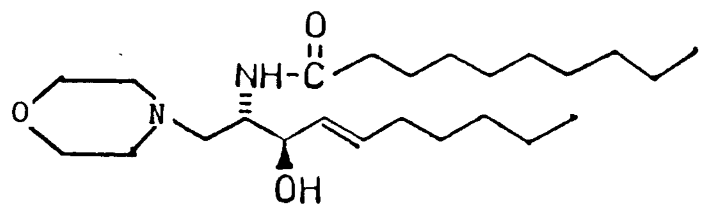

7. Ceramidderivate der allgemeinen Formel

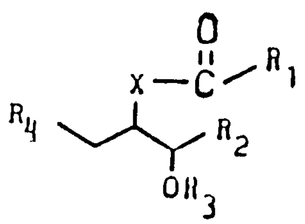

in der die einzelnen Reste folgende Bedeutungen haben:

X = NH oder $CH_2$,
$R_1$ = H oder $C_{1-11}$-Alkyl,
$R_2$ = $C_{2-9}$-Alkyl oder $C_{2-9}$-Alkenyl,
$R_3$ = H, $C_{1-6}$-Alkyl oder $C_{1-6}$-Acyl und
$R_4$ = OH, $C_{1-6}$-Alkoxy oder Morpholino

zur Anwendung als Inhibitoren der Sphingolipidsynthese.

8. Verbindungen nach Anspruch 1-6 zur Anwendung als Inhibitoren der Sphingolipidsynthese.

**Claims**

1. Ceramide derivatives of the general formula

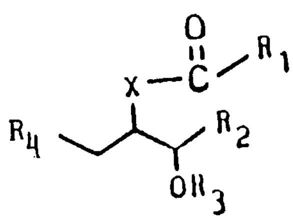

wherein the individual radicals have the following meanings:

X = -NH or -$CH_2$,
$R_1$ = H or $C_{1-11}$-alkyl,
$R_2$ = $C_{2-9}$-alkenyl wherein the double bond is in $\alpha$ position to the $OR_3$ group,
$R_3$ = H, $C_{1-6}$-alkyl or $C_{1-6}$-acyl and
$R_4$ = OH, $C_{1-6}$-alkoxy or morpholino.

2. Compounds according to claim 1, characterized in that the individual radicals have the following meanings:

X = NH,
$R_1$ = $C_{1-8}$-alkyl,
$R_2$ = $C_{3-6}$-alkenyl and

$R_3$ = H, methyl or acetyl and
$R_4$ = OH, $OCH_3$ or morpholino.

**3.** Compounds according to claim 1 or 2, characterized in that the double bond of the alkenyl radical in $R_2$ has the trans-configuration.

**4.** Compounds according to claim 1 or 2, characterized in that the double bond of the alkenyl radical of $R_2$ has the cis-configuration.

**5.** Compound according to claim 1 having the following formula:

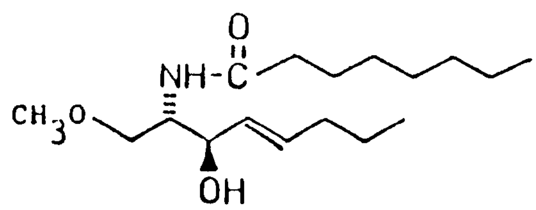

**6.** Compound according to claim 1 having the following formula

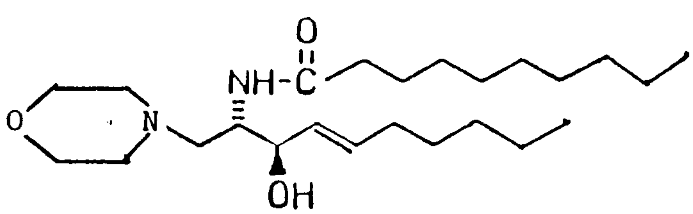

**7.** Ceramide derivatives of the general formula

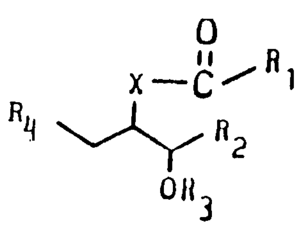

wherein the individual radicals have the following meanings:
X = -NH or $-CH_2$,
$R_1$ = H or $C_{1-11}$-alkyl,
$R_2$ = $C_{2-9}$-alkyl or $C_{2-9}$-alkenyl,
$R_3$ = H, $C_{1-6}$-alkyl or $C_{1-6}$-acyl and
$R_4$ = OH, $C_{1-6}$-alkoxy or morpholino for use
as inhibitors of sphingolipid synthesis.

**8.** Compounds according to claims 1 to 6 for use as inhibitors of sphingolipid synthesis.

## Revendications

1. Dérivés de céramide de formule générale

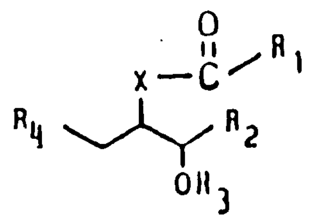

dans laquelle les différents restes ont les significations suivantes:
X = NH ou $CH_2$,
$R_1$ = H ou alkyle en $C_1$-$C_{11}$,
$R_2$ = alcényle en $C_2$-$C_9$, la double liaison se trouvant en position $\alpha$ par rapport au groupe $OR_3$,
$R_3$ = H, alkyle en $C_1$-$C_6$ ou acyle en $C_1$-$C_6$ et
$R_4$ = alcoxy en $C_1$-$C_6$ ou morpholino.

2. Composés selon la revendication 1, caractérisés en ce que les différents restes ont les significations suivantes:
X = NH,
$R_1$ = alkyle en $C_1$-$C_8$,
$R_2$ = alcényle en $C_3$-$C_6$,
$R_3$ = H, méthyle ou acétyle et
$R_4$ = $OCH_3$ ou morpholino.

3. Composés selon la revendication 1 ou 2, caractérisés en ce que la double liaison du reste alcényle dans le cadre de $R_2$ possède la configuration trans.

4. Composés selon la revendication 1 ou 2, caractérisés en ce que la double liaison dans le cadre de $R_2$ possède la configuration cis.

5. Composé selon la revendication 1, ayant la formule suivante:

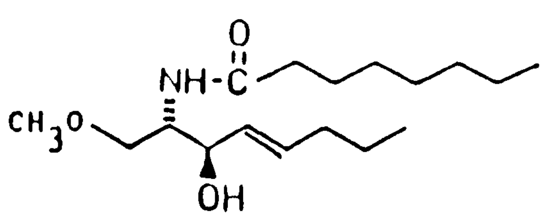

6. Composé selon la revendication 1, ayant la formule suivante:

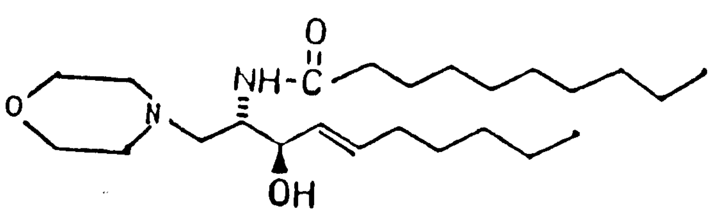

7. Dérivés de céramide de formule générale

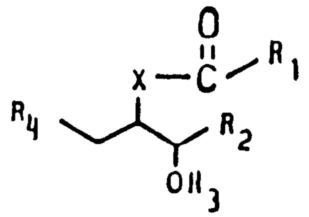

dans laquelle les différents restes ont les significations suivantes:

X = NH ou $CH_2$,
$R_1$ = H ou alkyle en $C_1$-$C_{11}$,
$R_2$ = alkyle en $C_2$-$C_9$ ou alcényle en $C_2$-$C_9$,
$R_3$ = H, alkyle en $C_1$-$C_6$ ou acyle en $C_1$-$C_6$ et
$R_4$ = OH, alcoxy en $C_1$-$C_6$ ou morpholino

pour l'utilisation comme inhibiteurs de la synthèse des sphingolipides.

8. Composés selon les revendications 1-6 pour l'utilisation comme inhibiteurs de la synthèse des sphingolipides.

Fig. 1

Fluorographie einer Dünnschichtelektrophorese

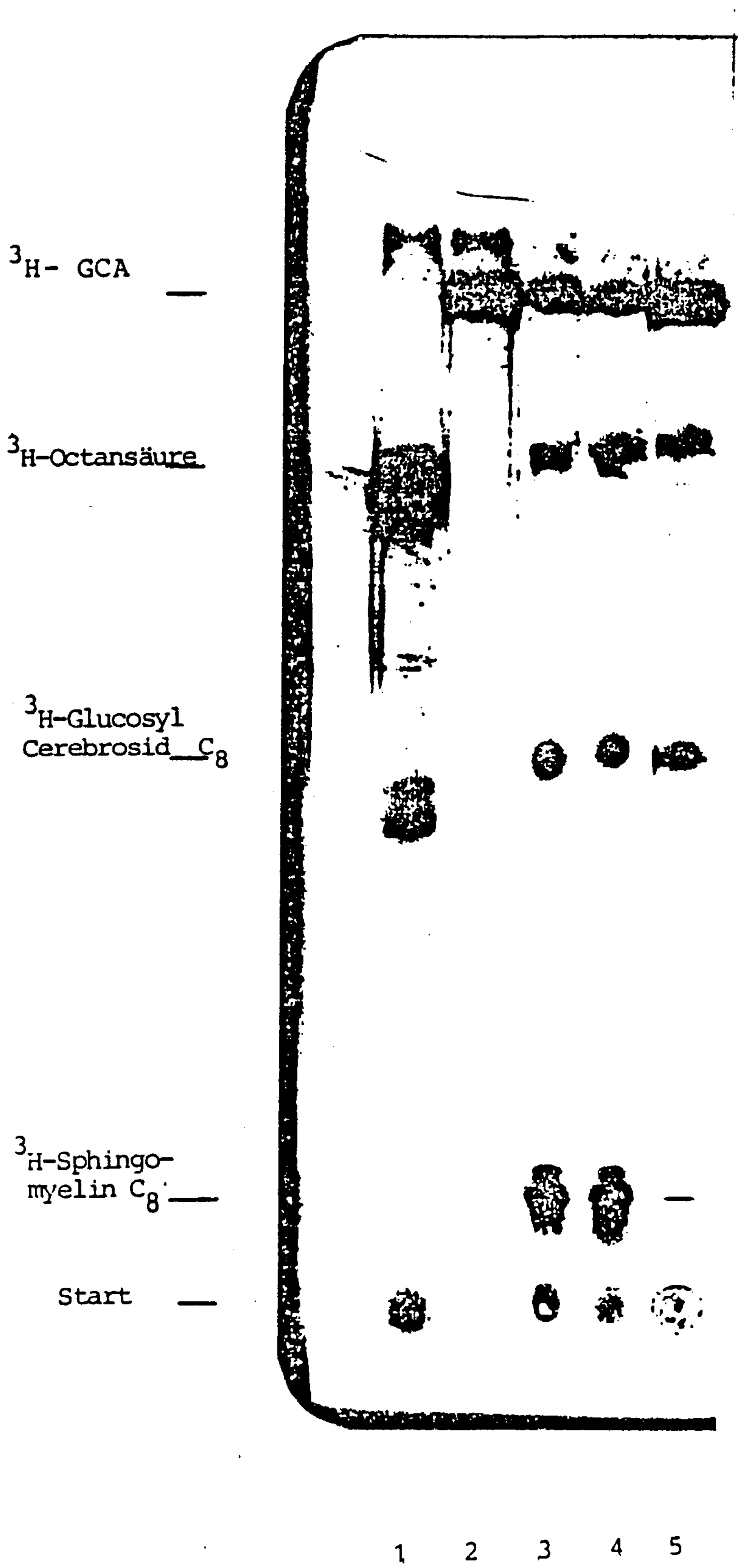

Fig. 2A

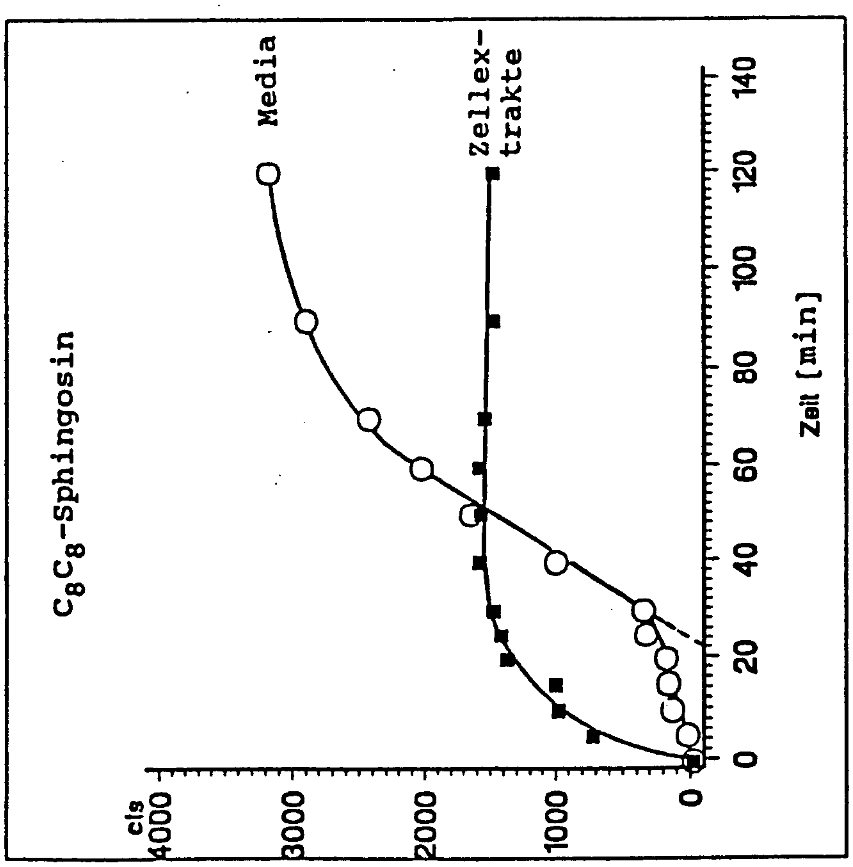
$C_8C_8$-Sphingosin
cts
4000
3000
2000
1000
0
0
20
40
60
80
100
120
140
Zeit [min]
Media
Zellex-
trakte

Fig. 2B

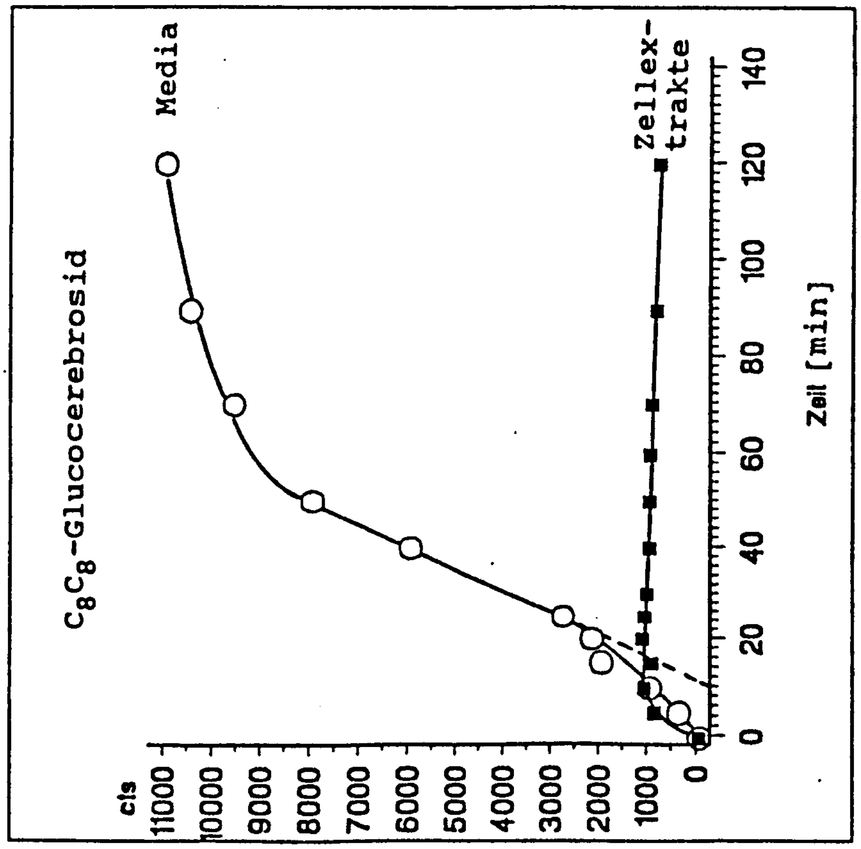
$C_8C_8$-Glucocerebrosid
cts
11000
10000
9000
8000
7000
6000
5000
4000
3000
2000
1000
0
0
20
40
60
80
100
120
140
Zeit [min]
Media
Zellex-
trakte

Fig. 3

A: Hemmung in intakten Golgi-Membranen

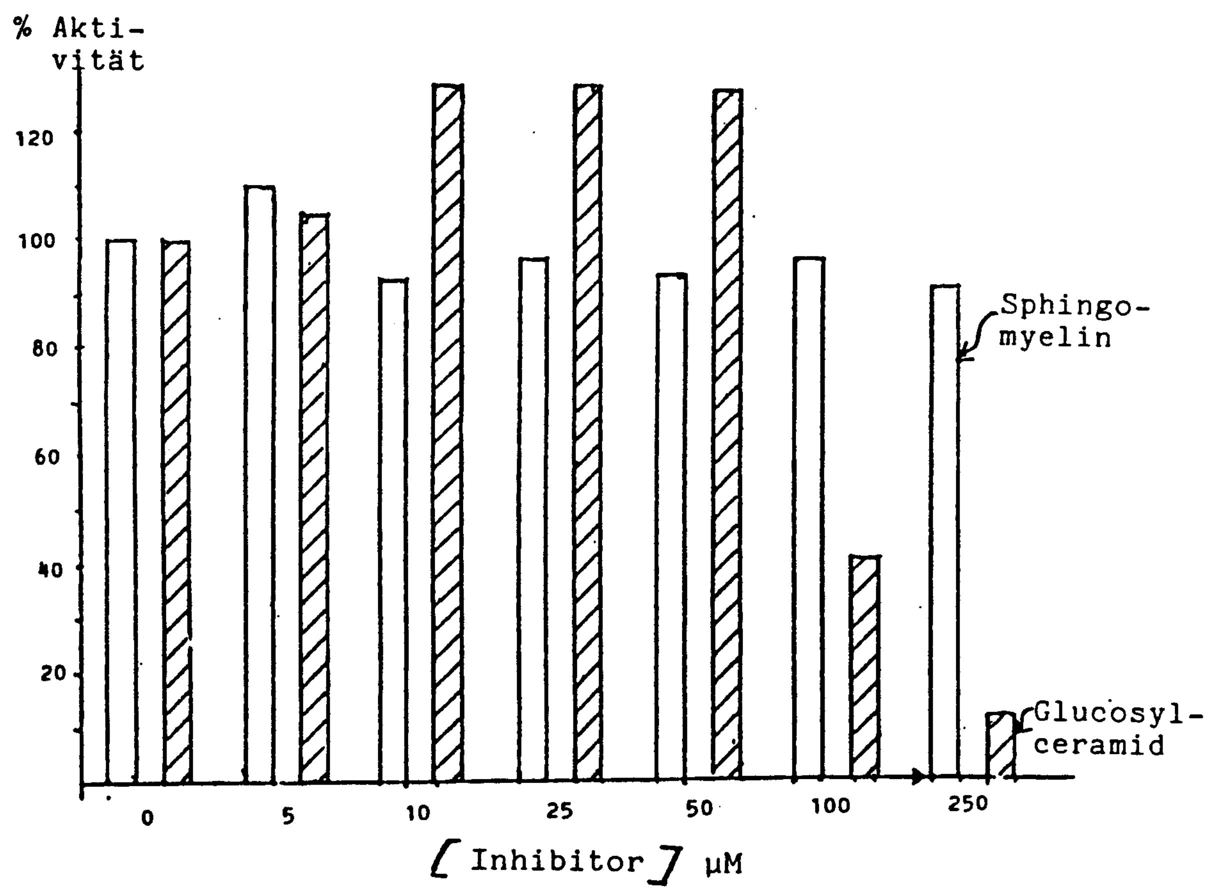

Fig. 4

B.: Hemmung in intakten Zellen: Medium

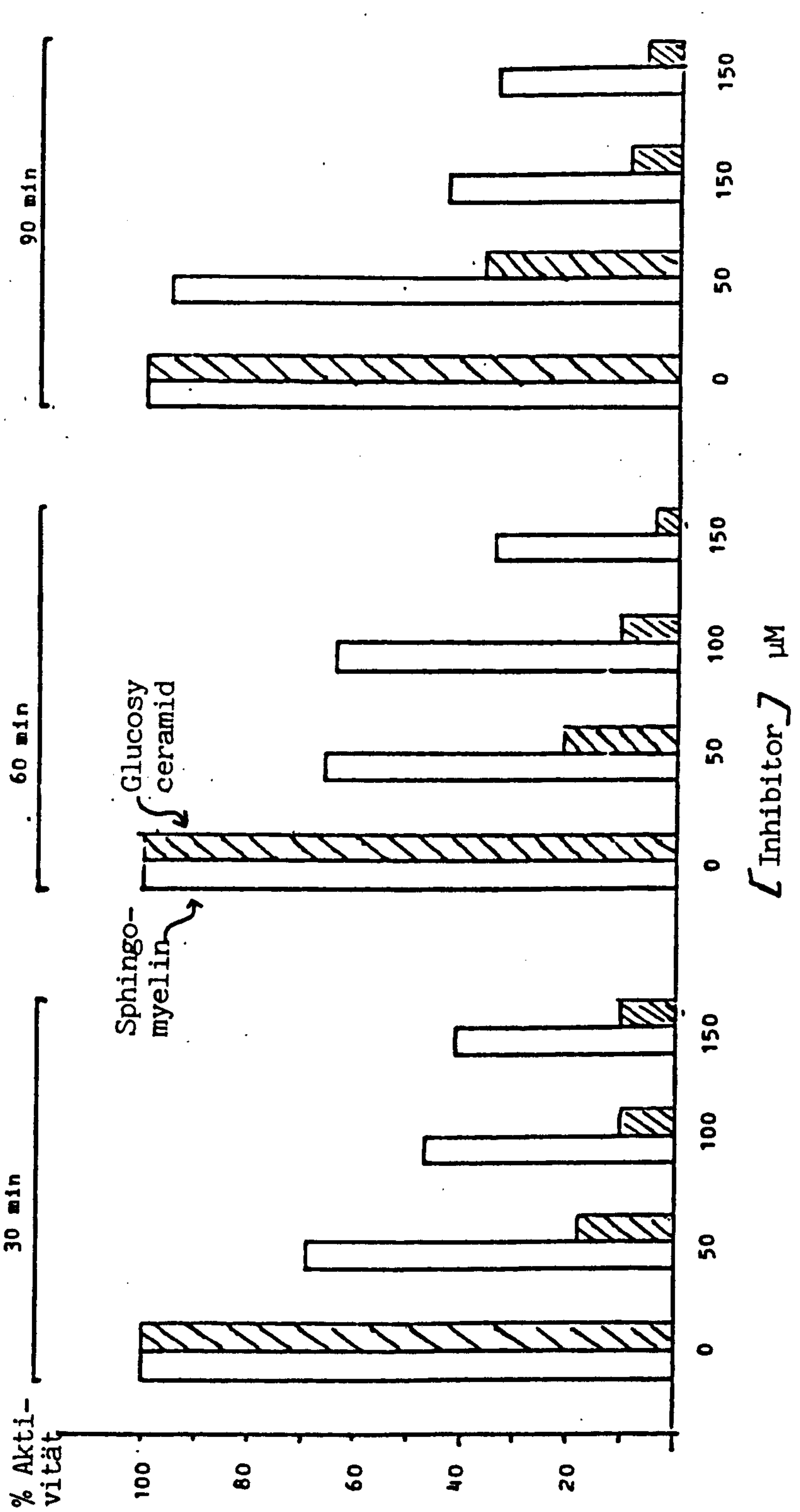

Fig. 5

B.: Hemmung in intakten Zellen: Zellextrakte